# EUROPEAN PATENT SPECIFICATION

(11) **EP 3 980 096 B1**
(45) Date of publication and mention of the grant of the patent: **13.12.2023**
(21) Application number: 20736430.8
(22) Date of filing: 08.06.2020
(51) Int. Cl.: A61M 5/142, A61M 5/168

(54) **BIOCOMPATIBLE POLYFUNCTIONAL CARRIER DEVICE, SYSTEM FOR CARRYING OUT FUNCTIONS WITHIN ORGANIC FLUIDS AND/OR ENVIRONMENTS**
BIOKOMPATIBLE MEHRFUNKTIONELLE TRÄGERVORRICHTUNG, SYSTEM ZUR DURCHFÜHRUNG VON FUNKTIONEN IN ORGANISCHEN FLUIDEN UND / ODER UMGEBUNGEN
DISPOSITIF DE SUPPORT POLYFONCTIONNEL BIOCOMPATIBLE, SYSTÈME POUR RÉALISER DES FONCTIONS DANS DES FLUIDES ET/OU DES ENVIRONNEMENTS ORGANIQUES

(30) Priority: 10.06.2019 IT 201900008508
(43) Date of publication of application: 13.04.2022
(73) Proprietor: Sleng S.r.l., 42121 Reggio Emilia (IT)
(72) Inventor: CAPPELLI, Fabio Renato, 40068 San Lazzaro di Savena Province of Bologna (IT); SACCARDI, Pierluigi, 42030 Vezzano sul Crostolo Province of Reggio Emilia (IT)
(74) Representative: Mola, Edoardo
(86) International application number: PCT/IB2020/055367
(87) International publication number: WO 2020/250107

(56) References cited:
- US-A1- 2006 165 805
- US-A1- 2015 157 837
- US-A1- 2016 030 721
- US-A1- 2018 256 816

## Description

The present invention concerns a polyfunctional carrier device composed of biocompatible and substantially biodegradable material suited to be carried within organic fluids and/or environments for carrying out at least one operative function, a system for carrying out at least one operative function within organic fluids and/or environments and employing for the purpose one or more polyfunctional carrier devices according to the invention.

The application field of the invention is the generic field of biological environments where it is possible and/or necessary to operate in a minimally invasive manner to carry out operations and functions within organic fluids and/or environments such as, for example, monitoring biological environments within living organisms as well as carrying out functions suited to direct and treat biological and/or physiological processes. To be suitable for these applications the devices must refer to nanoparticles engineering and deal with all the related constraints and technical problems of this field; the invention has been designed in particular for applications in the field of medicine, for example for the administration of medicaments, substances and remedies directly inside parts and/or organs to be treated through which blood and/or other vital fluids flow.

Document US2018/256816 A1 teaches about implantable drug delivery device produced using hydrogel MicroElectroMechanical (MEMS) devices. The subject matter of this document deals with delivery of substances in vivo thus including a reservoir and means to control the delivery. This approach can be efficient where there is the possibility to implant the device close to the tissue to be treated but less useful in case there if the need to deliver the substances such as medicaments, as well as substances and functions directly inside the human body only possible through the vehiculation by means of organic fluids.

Document US2015/157837 A1 also relates to the field of drug delivery devices employing catheters and/or cannulas to transport fluid from a reservoir to a patient and, more particularly, to a device for introducing a drug into a patient's bodily fluid, such as, for example, into perilymph in the human ear, as well as to methods for infusing drugs into cochlea for treatment of hearing loss and other disorders of hearing and vestibular function. Even if in the same field, the invention, similarly to US2018/256816, is not related to nanodevices for delivering substances by directly reaching internal organs of the human body.

Document US2016/030721A1 teaches about one or more pharmaceutically effective compounds stabilized in a sugar glass composition, at least one of the one or more stabilized pharmaceutically effective compounds in the sugar glass composition enclosed within the one or more compartments; and one or more reservoirs configured to provide access for one or more release agents to an interior of the sugar glass composition, wherein the one or more reservoirs are configured to controllably dispense the one or more release agents to disrupt the sugar glass composition from the interior of the sugar glass composition. One single device can embed one or more reservoirs and means to control the release are provided.

Document US2006/165805A1 relates to magnetic pole matrices and method of use thereof for tissue engineering and targeting systematic therapy for cardiovascular disease using magnetic polymer nanoparticles gene/drug delivery. The invention introduces magnetic pole matrices suitable for driving pharmaceutics compound in the form of magnetic bends inside biological body introducing limitations related to the need for magnetization of the substance to be driven to the target location e.g. inside veins.

In the field as above specified, the writer has no knowledge of the existence of any polyfunctional carrier device as indicated above, or any system similar to the one specified and described below in the present document.

Therefore the writer, the first in the field, has identified and implemented the possibility of exploring, treating and taking care organs and parts of biological systems in general, more specifically of the human body through the vehiculation of devices, such as medicaments, as well as substances and functions directly inside said organs and systems on which to intervene.

One object of the invention is to implement applicative and curative functions directly inside the organs and systems concerned with minimum possible impact and substantially in a non-invasive manner for the concerned organs and systems.

One object is to convey and implement a plurality of functions within organic fluids and/or environments such as, for example, visual monitoring of their state, electric stimulation of organs or "firing", the release of substances and remedies directly in a specific point through the implementation of a system according to the invention.

To achieve said objects, the subject of the present invention is a polyfunctional carrier device composed of biocompatible and substantially biodegradable material suited to be conveyed within organic fluids and/or environments for carrying out at least one operative function, and a system for carrying out at least one operative function within organic fluids and/or environments according to the specifications of the attached claims.

Advantageously, polyfunctional carrier devices according to the invention are able to move, adapt and assume precise established configurations within organic fluids and/or environments according to the system illustrated, in particular oriented and guided by means for the generation of a magnetic field, so that they have maximum adaptability and operativity within said organic fluids/environments. Within the latter they are able to simply and efficiently perform the required operative functions.

The polyfunctional carrier devices consist advantageously of a simple, efficient, reliable, biocompatible and substantially biodegradable structure within the organic fluids and/or environments in which they operate. Therefore, said organic fluids and environments are able to receive and dispose of said carrier devices without any harmful consequence for the health and biological functions thereof.

Furthermore, the polyfunctional carrier devices are simple to produce, highly customisable and flexible in their ability to carry out and implement a plurality of different operative functions in a simple and rational manner.

The system for carrying out at least one operative function within organic fluids and/or environments is simple and reliable.

Further objects, characteristics and advantages of the present invention will become clear from the following detailed description of a plurality of preferred embodiments of the invention and relative variations, provided purely for explicatory non-limiting purposes with the aid of the attached figures, therein:
- Figures 1 and 2 show respectively a plan view and a lateral elevation view of a first representation of a polyfunctional carrier device according to the invention, where in figure 2 it is in a particular operating condition and where furthermore the elevation view of a first embodiment of a system for carrying out at least one operative function within organic fluids and/or environments according to the invention is schematically illustrated.
- Figures 3 and 4 show respectively a plan view and a lateral elevation view of a second representation of a polyfunctional carrier device according to the invention, in particular during an initial step of preparation of the same and in a particular operating condition.
- Figures 5 and 6 illustrate the second representation of figures 3 and 4 in a final step of preparation of the same, in a plan view and lateral elevation schematic view respectively.
- Figures 7 and 8 illustrate schematic lateral views of a pair of details relative to the polyfunctional carrier device of figures 5 and 6.
- Figures 9 and 10 illustrate plan views respectively of a first and second variation of the polyfunctional carrier device of figures 5 and 6.
- Figure 11 shows an overhead perspective view of part of a system for carrying out at least an operating function within organic fluids and/or environments according to the invention, in particular a possible implementable spatial arrangement of a set of polyfunctional carrier devices according to the invention.
- Figures 12 and 13 show schematic views of an initialization step of the spatial arrangement of figure 11, a plan view and an overhead perspective view respectively.
- Figures 14 and 15 show respectively a lateral schematic view and a plan view of a system for carrying out at least an operative function within organic fluids and/or environments according to the invention, in particular with application on a human body.

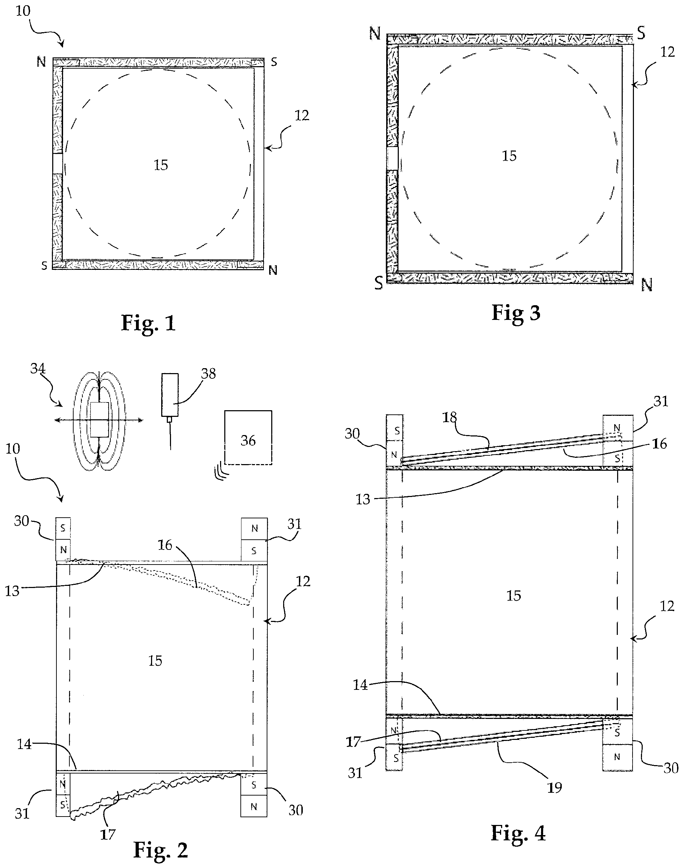

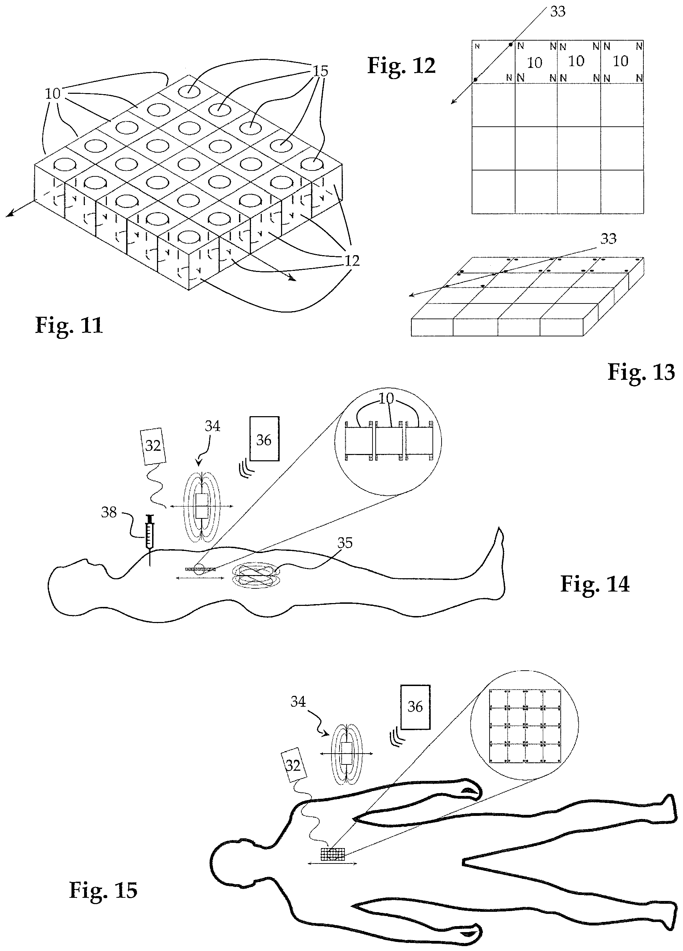

With reference in particular to figures 1-6, a polyfunctional carrier device consisting of biocompatible and substantially biodegradable material suited to be carried within organic fluids and/or environments for carrying out at least an operative function is indicated overall by 10. In further detail, the polyfunctional carrier 10 is composed of a main body 12 having the following characteristics:
- it consists of biocompatible and biodegradable material, in particular organic material having a certain rigidity and mechanical properties suited to providing a supporting structure such as, for example, a PHA polyester polymer as well as PGA polyglycolic acid or other organic materials having equivalent mechanical characteristics;
- it is shaped so as to have an outer form suited to allowing the aggregation of several carrier devices 10 in reciprocal contact at least with one face, as illustrated in figures 11-13, for example assuming a parallelepipedal shape having substantially similar overall dimensions;
- it is structured so as to define an inner space, in the example a substantially cylindrical shape, suited to constituting a reservoir 15 for a substance to be administered and provided with at least an opening, in detail a pair of openings 13 and 14 obtained in the area of the bases having substantially cylindrical shape and defining a pair of surfaces 13 and 14, external and opposite each other, for outflow of the substance to be administered towards the organic fluids and/or environments within which the carrier device 10 is conveyed;
- it has external dimensions ranging from nanometric to millimetric size, thus it can be simply carried in a few drops of fluid and in fairly small narrow organic environments such as, for example, the capillaries of a human body.

In a first representation of the invention, the following are associated with the main body 12 of the carrier device 10:
- a covering of biocompatible and biodegradable material for the pair of openings 13 and 14 suited to define the reservoir 15 for the substance to be administered, the covering being fitted and associated with the main body 12 so as to guarantee the seal of the reservoir 15 as well as to allow the opening thereof when activated by actuator means provided for the purpose, for example a pair of first sheets 16 and 17 made of polypropylene PP or other polymer with low expansion coefficient, shown in figures 2 and 4 are in the open work position for the outflow of the substance to be administered;
- magnetic means 30, 31, in particular of ferromagnetic or ferrimagnetic type, associated with each outer surface 13, 14 of the main body 12 and suited overall to generate a defined, polarized and directed magnetic field for guiding and positioning said carrier 10 within organic fluids and/or environments by magnetic fields operating outside said organic fluids and/or environments, and to aggregate a plurality of carried devices 10 in a defined spatial configuration such as, for example, a plate configuration as illustrated in figures 11-13.

In a second representation of the invention of figures 3-6, the following are furthermore associated with the main body 12 of the carrier device 10:
- a further covering of biocompatible and biodegradable material for the pair of openings 13 and 14 suited to operate as a support surface, indicated with 20 in its whole, for means and devices suited to implement at least an operative function of the polyfunctional carrier 10 within the organic fluid and/or environment where it operates, for example a pair of second sheets 18 and 19 composed substantially of the same material as the first sheets 16 and 17 and on which said means and devices suited to implement at least an operative function are associated. In particular, the second sheets 18 and 19, which operate in the example cited as support surfaces 20, are perfectly adherent and bound to the first sheets 16 and 17, for example by gluing.

As regards the magnetic means, while it is possible to use inorganic ferromagnetic materials, for example magnetized before their application on the main body 12, for the purpose of biocompatibility and substantial biodegradability of the carrier device 10, components made of organic materials can be used, obtained for example from the polymerization of products such as PANICNQ as well as TDAE-C60 or other equivalent materials. The magnetic means can be produced and polarized during manufacturing and subsequently applied to the polyfunctional carrier devices 10.

More in detail in the example illustrated, the magnetic means are of a first type 30 having S/N polarity and a second type 31 having N/S polarity oriented upwards with respect to the main body 12; said types alternate at the four corners of the outer surfaces 13 and 14. With said configuration a plurality of carrier devices 10 are naturally oriented and compacted, adhering to respective magnetically polarized faces of their main body 12, as illustrated for example in the plate arrangement of figures 11-13.

The said magnetic means of first type 30 and second type 31 are able, for example, to be obtained by polarizing, as required, an attested material being chosen to form the magnetic means through a magnetic field polarized prior to their application in the required configuration, for example by gluing, hence they are already polarized at the moment of application on the main body 12.

Furthermore it is possible to follow a polarization method of the magnetic means as illustrated in figures 11-13, where a plurality of carrier devices 10 are produced and processed from a plurality of relative main bodies 12 obtained from a single substantially plate-shaped solid body which is subsequently perforated by appropriate machines to form the reservoirs 15, as illustrated in figure 11. Subsequently, the plate is cut to form a plurality of main bodies 12 having definitive prismatic shape, which are then fitted with the coverings 16-20 depending on the type of carrier device to be made and, if necessary, with a substance to be dispensed filling the reservoir 15, as previously described. Later, the above-mentioned magnetic means are applied on the main body 12, for example at the four upper and lower corners of the prism, which initially do not present any magnetic polarization. It is now possible to polarize said magnetic means, through the presence of a strong polarizing external magnetic field, for example by making them all of the second type 31 with N/S polarity oriented upwards.

In order to overall generate on each carrier device 10 a defined, polarized and directed magnetic field, it is possible to carry out a magnetic depolarization operation which is performed, for example, through the emissions 33 of a 45° cross operated laser 32 as shown, as well as with beams at 90° with respect to the orientation of the carrier devices 10, so that the action of the laser beam intercepts and exceeds the Curie temperature in the magnetic means struck by the emissions 33, which are depolarized. Through the study of given paths, particular geometries can be studied to be assumed by the laser, thus depolarizing the carrier devices 10 in a particular way to achieve a required magnetic configuration in order to then create aggregations of carrier devices as desired.

In order to overall develop for each polyfunctional carrier device 10 a defined, polarized and directed magnetic field for guiding and positioning said carrier 10 within organic fluids and/or environments, the magnetic means of one type have greater magnetic intensity than those of the other type, in the example those of the second type 31 have greater intensity than those of the first type 30.

With specific reference to the first representation of the invention of figures 1-2 and as further illustrated in figures 14-15, a system is illustrated for carrying out at least an operative function within organic fluids and/or environments according to the invention, comprising:
- at least a polyfunctional carrier device 10 as described;
- injection means for injecting polyfunctional carrier devices 10 into organic fluids and/or environments, for example a syringe 38 inside which a physiological solution is prepared containing said devices 10;

- generator means for generating a magnetic field of a type suited to directing the polyfunctional carrier devices 10 into the organic fluid and/or environment, operating according to given and desired spatial paths, for example a permanent magnet 34;
- control devices for carrying out at least a function of the polyfunctional carrier 10 within organic fluids and/or environments, in detail generator means for generating ultrasounds 36 of a type suited to creating a shock wave of a certain pressure in order to move and/or perforate and to cause collapse of the first sheets 16 and 17, thus freeing the content of the reservoir 15 and allowing it to flow out into the organic fluid and/or environment where the polyfunctional carrier devices 10 operate, as illustrated in figure 2.

Therefore, at the moment when the ultrasound generator 36 operates, emitting a certain ultrasound frequency and power suited to control the substantial opening of the pair of openings 13 and 14 through substantial collapse of the first sheets 16 and 17 as described above, the same first sheets 16 and 17 constitute actuation means for implementing at least a function of the polyfunctional carrier 10 within organic fluids and/or environments. In this representation of the invention, the function concerns administration of the content of the reservoir 15 of the carrier device 10 by outflow of the same through the openings 13, 14 towards the organic fluids and/or environments where it is operating. In greater detail, the carrier device 10 in association with other carrier devices 10 such as, for example, illustrated in figures 11-13, is oriented and positioned in a desired point by the action of the magnetic field generator means, namely the permanent magnet 34 which moves spatially so as to guide the carrier devices 10 to points where the only function possible for this type of configuration, namely the administration of a substance contained in the respective reservoirs 15, is implemented.

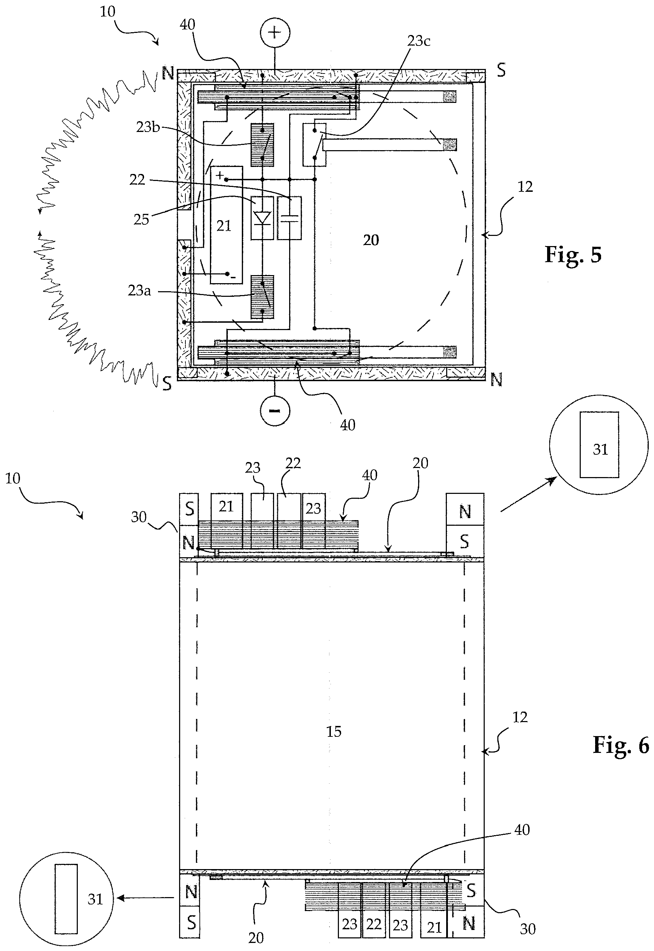

Figures 3 and 4 illustrate a polyfunctional carrier device 10 in a first step of production and preparation of the same, in which the following operations have been carried out:
- shaping the organic material of which the main body 12 is composed according to an outer form suited to allow the aggregation of several carrier devices 10 in reciprocal contact at least with a respective face, and the creation of a through hole to define the main shape of the reservoir 15,
- covering the openings 13 and 14 with the first sheets 16 and 17, securing them, for example by means of gluing, to the edges of the main body 12 thus determining the entire volume of the reservoir 15 after it has been filled with a substance to be administered,
- causing to adhere, for example by means of gluing, on the first sheets 16 and 17 the second sheets 18 and 19 of which at least one (in figures 5-6 and 9-10 both) operating as a support surface 20 for means and devices suited to implement at least an operative function of the polyfunctional carrier device 10.

In figure 4, the second sheets 18 and 19 are illustrated in the operative condition of opening of the reservoir 15 in order to carry out the function of administration of a substance contained in it; said opening is towards the outside as illustrated and described in the continuation of the present description.

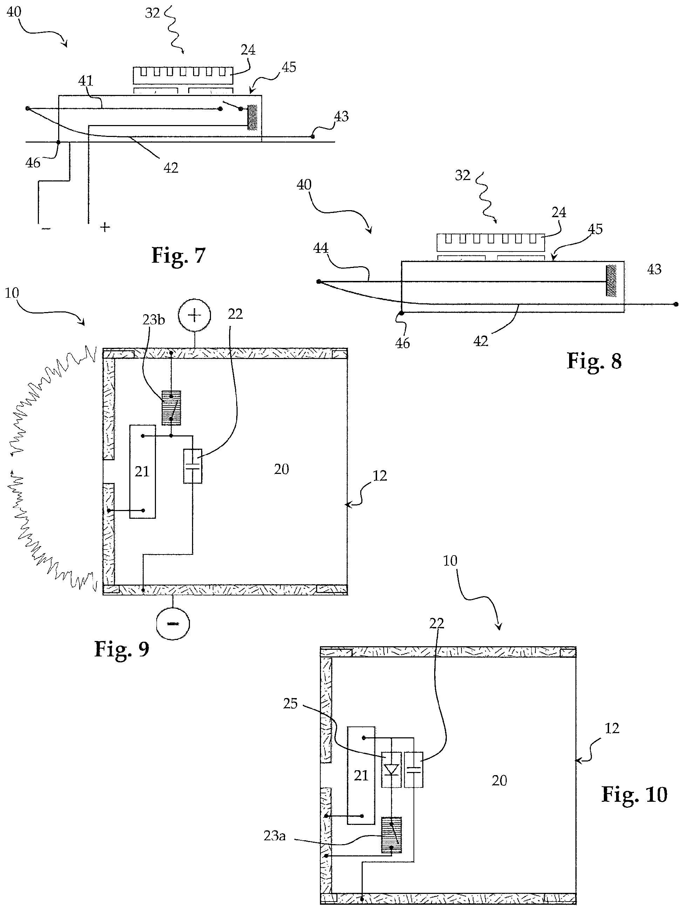

The support surfaces 20 are well detailed and can be seen in the successive figures 5-6 and respective variations 9-10, which will be described below with reference to the same figures, and to figures 7 and 8 which illustrate some details thereof.

On the support surfaces 20 the following are associated in a fixed manner and adhering to the same:
- means for powering and/or storing an electric charge, for example a solar panel made of biocompatible and substantially biodegradable material or OSC 21 suited to guarantee continuous electric recharge when exposed to the light, which is in electric connection with a capacitor made of biocompatible and substantially biodegradable material or OCAP 22 having the function of standby battery and/or rapid discharge of the energy stored in the solar panel 21;
- actuation means for carrying out at least a function of the polyfunctional carrier 10 within organic fluids and/or environments, for example at least an electric switch 23a, 23b consisting preferably of biocompatible material such as for example polyaniline PAMI, polypyrrole PPY or equivalent materials and operated by a plasmonic filter 24, visible in figures 7 and 8, which switches the switch 23 to the closed position ON when it is activated by a laser beam at a given emission frequency to which actuation of the same filter 24 is set, as well as a switch 23c operated by ultrasounds.

The actuation means of at least a function of the polyfunctional carrier 10 include the following devices which use the electric current stored in the power supply and/or storage means 21, 22 and are electrically connected to them:
- an oled made of biocompatible and substantially biodegradable material 25 with the function of tracing and detecting the position in order to effectively and accurately detect the area where the carrier devices 10 operate;
- a "+" and "-" polarity pair obtained on the lateral surface of the main body 12 of the carrier device 10 which consist of organic material, for example PEDOT:PSS, in order to carry an electric stimulation or firing function on some parts of the organic environment in which the carrier devices operate;
- an opening device for opening the tank 15, indicated overall by 40 and of types as detailed below with reference to figures 7-8.

The power supply and/or storage means of an electric charge 21, 22 are associated with the main body 12 through the support surfaces 20 and are furthermore electrically connected to the actuation means 21-25, 40, +,- to carry out at least a function of the polyfunctional carrier 10 inside the organic fluids and/or environments where they will operate, for example in the blood in a human body for treatments and in order to avoid invasive surgical interventions.

The various means, devices and circuits present on the support surfaces 20 as illustrated in the second embodiment of the invention of figures 5 and 6 and relative variations of figures 9 and 10 are detailed below; in these variations only one of the functions of the more complex form of figures 5 and 6 is present.

With reference to the function of tracing and detection of the position by one or more functional carrier devices 10 aggregated together if necessary, the solar panel 21 is electrically connected in parallel with the capacitor 22 and with the oled 25, where the switch 23a is connected to the oled 25 so that, once in the closed position ON activated by a given activation frequency emitted by a laser device 32 as schematically illustrated in figures 14-15, it allows powering and therefore switch-on of the oled 25 by the capacitor 22 and/or the solar panel 21.

With reference to the function of electric stimulation or firing on some parts of the organic environment in which the carrier devices operate, the solar panel 21 is connected in parallel to the capacitor 22 so that the switch 23b powers, in the same way described previously when activated by a laser device 32 at a frequency different from the previous one, the "+" and "-" polarity pair permitting the activation of an electric discharge from the capacitor 22 which initiates the desired electric stimulation.

With reference to the opening function of the reservoir 15, the solar panel 21 is electrically connected in parallel with the capacitor 22 and with the opening device 40 of the reservoir 15, which can also have inside it a switch with plasmonic filter 24 as specified further on, so as to allow power supply and actuation once the circuit is closed. Furthermore, the opening device 40 is electrically connected to an ultrasound switch 23c able to control the actuation also by means of ultrasounds, so that functioning continues also in operating situations in which the laser devices can no longer operate.

Advantageously, the presence of an ultrasound switch allows the carrier device to release and diffuse the substance present in the reservoir 15 in all the areas where a laser beam is not able to penetrate with its light frequencies, thus ensuring greater effectiveness and flexibility of the system according to the invention.

With reference to figures 7 and 8, the opening device 40 of the reservoir 15 and a variation thereof are schematically illustrated. The opening device 40 consists of a body 45 connected to the main body 12 of the carrier device 10 by means of a hinge point 46 able to rotate the entire body 45, inside which are the elements suited to perform the function of opening the reservoir 15 by lifting a flap of the support surface 20, namely of the first and second sheets 16-19 reciprocally glued to each other as illustrated in figure 4.

In the case of figure 7 a switch with plasmonic filter 24 is present able to make electric current flow to the device 40 as previously described once a laser device 32 switches its state to position ON through the intervention of a laser beam at a given activation frequency thereof. Alternatively, an ultrasound switch 23c can be present with the same function activated this time by an emission of ultrasounds with given intensity and frequency on which the switch is calibrated.

The closure of the switch in the ON position causes the electric current coming from the solar panel 21 and/or from the capacitor 22 to power a bar of inorganic bistable material, for example NiTiNol 41, connected at one end to the body 45 of the carrier device 10 and at the other end to an end of a shaped part 42 consisting of organic material with low thermal deformation coefficient, for example polypropylene PP or equivalent materials. The shaped part 42 is then securely connected, for example by gluing, with one end in a point 43, to the support surface 20. Due to the heat emanated from laser devices 32 and closure of the plasmonic filter switch 24, the NiTiNol bar 41 heats up, thus significantly increasing its length, dragging with it horizontally the shaped part 42 which expands much less due to the nature of its materials, thus allowing the body 45 to rotate around the hinge point 46 raising the distal flap of the support surface 20, therefore the second sheets 18 and 19 and the first sheets 16 and 17 glued to them, thus performing the function of opening the reservoir 15.

It is possible to re-close the reservoir 15, causing the support surface 20 to adhere to the upper edges of the same, by simply commanding another laser pulse which, switching again the plasmonic filter switch 24 to the ON position, heats again the NiTiNol bar 41 which, due to its nature, shortens, substantially returning to the same length it had at the beginning and dragging with it the entire body 45, which now rotates in the opposite direction round the point 46, closing the reservoir 15.

Advantageously, the use of a bar of inorganic bistable material makes the opening device 40 particularly reliable since this material has excellent mechanical capabilities in elongation and shortening, thus guaranteeing effective opening and closing of the support surface with relative delivery of the substance present in the reservoir.

Figure 8 illustrates a variation of the opening device 40 having structure very similar to the previous one, the differences being:
- the presence of a plasmonic filter 24 instead of a plasmonic filter switch 24 no longer necessary;
- instead of the bar of bistable material, typically inorganic, an actuator bar 44 is used consisting of biodegradable organic material with high thermal deformation coefficient such as, for example, polyethylene PE or other equivalent materials.

The operation of the device 40 is very similar to the previous one. For activation of the actuator bar 44 the sole energy of the laser device at a given frequency is sufficient; the latter passes through the plasmonic filter 24 and heats the same bar, which begins to elongate raising the second sheets 18 and 19 following the same procedures as those already described. In this case the support surface 20, namely the second sheets 18 and 19, automatically close on the reservoir 15 at the moment when the actuator bar 44 cools in contact with the fluids in which the carrier device 10 operates.

Advantageously, said solution allows wider biodegradability of the carrier device, not using any inorganic materials for performing the administration function, said materials being much more difficult to dispose of within an organism.

Means for screening and protection from the light, therefore also from the laser beams, are advantageously applied on the support surfaces 20, above the means for powering and/or storing an electric charge 21, 22 and the means for carrying out at least a function 21-25, 40, +, -; said screening and protection means are made of mainly organic material for example according to the known "Heat Mirror" technology, which entails overlap of biocompatible materials such as, for example, metallic silver, and biodegradable materials such as, for example, titanium dioxide. Said light screening and protection means are applied on all the components cited except for the plasmonic filters 24, so as to protect the above-mentioned means 21-25, 40, +, - from possible overheating and interferences that could occur due to the laser beams that impact the entire carrier device 10 and therefore the relative support surfaces 20.

Figures 14-15 further illustrate a system for carrying out at least one operative function within organic fluids and/or environments according to the invention in which the following are present:
- at least one polyfunctional carrier device 10, in particular associated with other carrier devices 10 in a configuration adapted to carry out all the functions provided for by the protocol such as, for example, a plate configuration as illustrated in figures 11-13;
- injection means for injecting polyfunctional carrier devices 10 into organic fluids and/or environments, for example a syringe 38 inside which a physiological solution is prepared containing said devices 10 to be injected inside a biological organism;
- generator means for generating a magnetic field of a type designed to direct the polyfunctional carrier devices 10 into the organic fluid or environment where they will operate according to given and desired spatial paths, for example a permanent magnet 34 as well as a sticking plaster 35 provided with magnets and applicable in the vicinity of the part on which to operate;
- control devices for carrying out at least a function of the polyfunctional carrier 10 within organic fluids and/or environments.

Said control devices comprise:
- generator means for generating ultrasounds 36 of a type suited both to create a shock wave with a certain pressure, which moves and/or perforates the first sheets 16 and 17 and any second sheets 18 and 19 of the carrier devices 10, and of a type suited to activate an ultrasound switch 23c;
- laser devices 32 suited to emit a series of light frequencies for the isolated or simultaneous activation of respective plasmonic filters 24 present in the plasmonic switches 23a, 23b and inside the opening device 40.

Below we describe the operation, perfectly intuitive, of the above system for carrying out at least an operative function within organic fluids and/or environments, implemented through the following steps:
- manufacture and preparation of a plurality of polyfunctional carrier devices 10 in configurations designed for carrying out a plurality of desired functions, which can be compacted and arranged in different ways according to the configuration selected for the magnetic means 30, 31 and how these means are fitted on the main body 12;
- injection of said polyfunctional carrier devices 10 within organic fluids and/or environments, for example through a syringe 38 inside which a physiological solution is prepared containing the devices 10;
- orientation and positioning of the carrier devices 10 injected according to the preceding step by means of movement and/or positioning of the magnetic field generator means 34, 35;
- activation of the control devices for carrying out at least a function of the carrier device 10 depending on the operation and/or treatment to be implemented via the system.

Therefore, the following can be selectively or together activated:
- the ultrasound generator means 36 able to create a shock wave suited to move and/or perforate the first sheets 16 and 17 and any second sheets 18 and 19 of the carrier devices 10 rather than to activate an ultrasound switch 23c;
- laser devices 32 suited to emit a series of light frequencies for isolated or simultaneous activation of respective plasmonic filters 24 able to implement, together with the respective switches 23a and 23b, the operative functions of the carrier devices 10 desired and previously described.

It is clear that, for a person skilled in the art, further numerous variations are possible to the polyfunctional carrier device composed of biocompatible and substantially biodegradable material designed to be carried within organic fluids and/or environments for carrying out at least an operative function, and to the system for carrying out at least an operative function within organic fluids and/or environments according to the invention; it is also clear that in its practical actuation the forms of the details illustrated can be different and can be substituted with technically equivalent elements.

For example, the main body of the polyfunctional carrier device can be shaped internally solid, namely without the cavity having the function of a reservoir. On the main body, at least a support surface can be applied, for example by means of gluing, bearing on it means and devices designed to implement at least an operative function of the polyfunctional carrier within the organic fluid and/or environment where it operates.

The actuation means of at least a function of the polyfunctional carrier can consist furthermore of a camera, pH sensors, temperature and/or pressure sensors, preferably consisting of organic, biocompatible and substantially biodegradable material.

It is possible to use a combination solely of ultrasound switches to activate the functions of the carrier devices differently from what is shown in the examples illustrated: for example, an ultrasound switch can be used to activate the electrical stimulation circuit illustrated in figure 9. Advantageously the carrier devices thus equipped can operate, in a biological organism, at much greater depth levels than when plasmonic filter switches are used, namely in all cases in which a laser beam cannot penetrate, or would penetrate but would burn the tissues of the biological organism it intercepts before activating the switch.

## Claims

1. A polyfunctional carrier device composed of biocompatible and substantially biodegradable material, suited to be conveyed within organic fluids and/or environments for carrying out at least an operative function, comprising:
- a main body (12), composed of biocompatible and biodegradable material, in particular suited to constitute a reservoir (15) for a substance to be administered and provided with at least an opening (13,14) for the outflow of a substance to be administered towards said organic fluids and/or environments;
- actuation means (16,17;21-25,40,+,-) of at least a function of the polyfunctional carrier (10) within organic fluids and/or environments,
**characterized in that** it comprises, associated with the main body (12), magnetic means (30,31) in the form of a plurality of magnetic elements of a first type (30) and of a plurality of magnetic elements of a second type (31) having respectively South/North and North/South polarity oriented upwards with respect to the main body(12), suited to assuming a defined magnetic polarity for guiding and positioning said carrier (10) within organic fluids and/or environments by magnetic fields operating externally to said organic fluids and/or environments, and are disposed along the outer surfaces (13,14) of the main body (12), suited overall to generating a magnetic field defined, polarized and directed in order to aggregate a plurality of carrier devices (10) in a spatial configuration defined by said magnetic means (30,31).

2. The polyfunctional carrier device, according to the preceding claim, **characterized in that** the main body (12) is shaped so as to have an outer form suited to allowing the aggregation of several carrier devices (10) in reciprocal contact at least with a face.

3. The polyfunctional carrier device, according to one of the preceding claims, **characterized in that** it comprises means for supplying and/or storing an electric charge (21,22) associated with said main body (12), the actuation means of at least a function (21-25,40,+,-) being associated with the main body (12) and with said means for supplying and/or storing an electric charge (21,22) for carrying out said at least one function within the organic fluids and/or environments where it operates.

4. The polyfunctional carrier device, according to the preceding claim, **characterized in that** it comprises, associated with the main body (12), at least a support surface (20) for means and devices suited to implementing at least an operative function of the polyfunctional carrier device (10) with which said means for supplying and/or storing an electric charge (21,22) and/or said means for actuating at least a function (21-25,40,+,-) are associated.

5. The polyfunctional carrier device, according to one of the claims 3-4, **characterized in that** the actuation means of at least one function comprise, selectively or together, the following devices and means:
- an OLED made of organic material (25) with the function of tracing and detecting the position;
- a polarity pair (+, -) obtained on the main body (12) of the carrier device (10) consisting of organic material in order to convey an electric stimulation function to some parts of the organic environment in which the carrier devices (10) operate;
- an opening device (40) for opening the reservoir (15);
being said devices controlled either:
- by an electric switch (23a,23b) made of biocompatible material and operated by the energy of a laser beam (32) filtered by a plasmonic filter (24), the electric switch (23a, 23b) suited to commute to the closed position when it is activated by the heat generated the incident laser beam at a given emission frequency to which the said filter (24) is calibrated or;
- by an ultrasound switch (23c) that can be operated by ultrasounds .

6. The polyfunctional carrier device, according to one of the claims 4-5, **characterized in that** it comprises means for screening and protection from the light applied above said support surfaces (20) and relative means and devices suited to implement at least an operative function of the polyfunctional carrier device (10) associated with it, in particular said means for supplying and/or storing an electric charge (21,22) and/or said means for carrying out at least a function (21-25,40,+,-), said means for screening and protection from the light not being present above any plasmonic filters (24) present on the support surfaces (20).

7. A system for carrying out at least an operative function within organic fluids and/or environments, **characterized in that** it comprises:
- at least one polyfunctional carrier device (10) according to one of the preceding claims;
- injection means (38) for injecting polyfunctional carrier devices (10) into organic fluids and/or environments;
- generator means for generating a magnetic field (34,35) of a type suited to directing the polyfunctional carrier devices (10) into the organic fluid and/or environment for operation according to given and desired spatial paths;
- control devices (36;32,36) for carrying out at least a function of the polyfunctional carrier (10) within organic fluids and/or environments.

8. The system for carrying out at least an operative function, according to the preceding claim, **characterized in that** the control devices comprise:
- ultrasound generator means (36) of a type suited both to creating a shock wave having a certain pressure suited to moving and/or perforating first sheets (16,17) of the carrier devices (10) thus releasing the substance to be administered contained in the reservoir (15), and to activating an ultrasound switch (23c);
- laser devices (32) suited to emitting a series of light beams at given emission frequencies for the isolated or simultaneous activation of one or more electric switches (23a, 23b), being the light beams filtered by plasmonic filters (24) calibrated on the said emission frequencies.

## Patentansprüche

1. Eine polyfunktionelle Trägervorrichtung, welche aus biokompatiblem und im Wesentlichen biologisch abbaubarem Material besteht und geeignet ist, in organischen Fluiden und/oder Umgebungen transportiert zu werden, um mindestens eine operative Funktion durchzuführen, die jeweils Folgendes umfasst:
- einen Hauptkörper (12), welcher aus biokompatiblem und biologisch abbaubarem Material besteht, der insbesondere geeignet ist, ein Speicherbecken (15) für eine anzuwendende Substanz zu bilden, das mit mindestens einer Öffnung (13, 14) für den Abfluss einer anzuwendenden Substanz in Richtung der genannten organischen Fluide und/oder Umgebungen ausgerüstet ist,
- Antriebselemente (16, 17; 21-25, +,-) von mindestens einer Funktion der polyfunktionellen Trägervorrichtung (10) in organischen Fluiden und/oder Umgebungen,
**dadurch gekennzeichnet, dass** sie in Verbindung mit dem Hauptkörper (12) Magnetelemente (30, 31) in Form einer Vielzahl von Magnetelementen eines ersten Typs (30) und einer Vielzahl von Magnetelementen eines zweiten Typs (31) umfasst, welche jeweils eine Süd/Nord- und Nord/Süd-Polarität aufweisen, die in Bezug auf den Hauptkörper (12) nach oben ausgerichtet und geeignet sind, eine bestimmte magnetische Polarität für die Führung und Positionierung der genannten Trägervorrichtung (10) in den genannten organischen Fluiden und/oder Umgebungen anzunehmen, und zwar über entsprechende Magnetfelder, welche außerhalb der genannten organischen Fluide und/oder Umgebungen arbeiten und entlang der äußeren Oberflächen (13, 14) des Hauptkörpers angeordnet sowie insgesamt geeignet sind, ein Magnetfeld zu erzeugen, welches bestimmt, polarisiert und ausgerichtet wird, um eine Vielzahl entsprechender Trägervorrichtungen (10) in einer räumlichen Konfiguration zu aggregieren, die durch die genannten Magnetelemente (30, 31) bestimmt wird.

2. Die polyfunktionelle Trägervorrichtung gemäß dem vorausgegangenen Anspruch, **dadurch gekennzeichnet, dass** der Hauptkörper (12) so ausgebildet ist, dass er eine äußere Form aufweist, welche geeignet ist, die Gruppierung mehrerer Trägervorrichtungen (10) in gegenseitiger Berührung von jeweils mindestens einer Seite zu gestatten.

3. Die polyfunktionelle Trägervorrichtung gemäß einem der vorausgegangenen Ansprüche, **dadurch gekennzeichnet, dass** sie Elemente für die Lieferung und/oder Speicherung einer elektrischen Ladung (21, 22) in Verbindung mit dem genannten Hauptkörper (12) umfasst, wobei die Antriebselemente mindestens einer Funktion (21-25, 40, +,-) mit dem Hauptkörper (12) und mit den genannten Elementen für die Lieferung und/oder Speicherung einer elektrischen Ladung (21, 22) zur Durchführung der genannten mindestens einen Funktion in den organischen Fluiden und /oder Umgebungen, verbunden sind, in denen sie arbeitet.

4. Die polyfunktionelle Trägervorrichtung gemäß dem vorausgegangenen Anspruch, **dadurch gekennzeichnet, dass** sie in Verbindung mit dem Hauptkörper (12) mindestens eine Tragfläche (20) für Elemente und Vorrichtungen umfasst, welche geeignet sind, mindestens eine operative Funktion der polyfunktionellen Trägervorrichtung (10) durchzuführen, mit der die genannten Elemente zur Lieferung und/oder Speicherung einer elektrischen Ladung (21, 22) und/oder die genannten Elemente zum Antrieb mindestens einer Funktion (21-25, 40, +, -) verbunden sind.

5. Die polyfunktionelle Trägervorrichtung gemäß einem der Ansprüche 3 - 4, **dadurch gekennzeichnet, dass** die Antriebselemente mindestens einer Funktion jeweils wahlweise oder zusammen folgende Vorrichtungen und Elemente umfassen:
- eine organische Leuchtdiode (OLED) aus organischem Material (25) mit der Funktion der Ermittlung und Feststellung der Position;
- ein Polaritätspaar (+, -) auf dem Hauptkörper (12) der Trägervorrichtung (10), welches aus organischem Material besteht, um eine elektrische Stimulation zu einigen Teilen der organischen Umgebung zu senden, in der die Trägervorrichtungen (10) arbeiten;
- eine Öffnungsvorrichtung (40) zur Öffnung des Speicherbeckens (15);
wobei die genannten Vorrichtungen jeweils folgendermaßen gesteuert werden:
- entweder durch einen elektrischen Schalter (23a, 23b), der aus biokompatiblem Material besteht und durch die Energie eines Laserstrahls (32) angetrieben wird, welcher durch einen plasmonische Filter (24) gefiltert wird, wobei der elektrische Schalter (23a, 23b), geeignet ist, auf die geschlossene Stellung umzuschalten, wenn er durch die Hitze eingeschaltet wird, welche durch den einfallende Laserstrahl bei einer bestimmten Emissionsfrequenz erzeugt wird, auf die der genannte Filter (24) jeweils geeicht ist, oder
- durch einen Ultraschallschalter (23c), der durch Ultraschall betätigt werden kann.

6. Die polyfunktionelle Trägervorrichtung gemäß einem der Ansprüche von 4 - 5, **dadurch gekennzeichnet, dass** sie Elemente zur Abschirmung und zum Schutz vor dem Licht oberhalb der genannten Tragflächen (20) sowie entsprechende Elemente und Vorrichtungen umfasst, welche geeignet sind, mindestens eine operative Funktion der damit verbundenen polyfunktionellen Trägervorrichtung (10) durchzuführen; insbesondere die genannten Elemente zur Lieferung und/oder Speicherung einer elektrischen Ladung (21, 22) und/oder die genannten Elemente zur Durchführung von mindestens einer Funktion (21 - 25, 40, +,-), wobei die genannten Elemente zur Abschirmung und zum Schutz vor dem Licht über keinen der sich auf den Tragflächen (20) befindenden plasmonischen Filtern (24) vorhanden sind.

7. Ein System zur Durchführung von mindestens einer operativen Funktion in organischen Fluiden und/oder Umgebungen, **dadurch gekennzeichnet, dass** es Folgendes umfasst:
- mindestens eine polyfunktionelle Trägervorrichtung (10) gemäß einem der vorausgegangenen Ansprüche;
- Einspritzelemente (38) für die Einspritzung polyfuktioneller Trägervorrichtungen (10) in organische Fluide und/oder Umgebungen;
- Generatorelemente zur Erzeugung eines Magnetfelds (34, 35) eines Typs, welcher geeignet ist, die polyfunktionellen Trägervorrichtungen (10) zwecks Betrieb in das organische Fluid und/oder die Umgebung zu leiten, und zwar jeweils gemäß vorgegebenen und erwünschten räumlichen Bahnen;
- Steuervorrichtungen (36, 32, 36) zur Durchführung von mindestens einer Funktion der polyfunktionellen Trägervorrichtungen (10) in organischen Fluiden und/oder Umgebungen.

8. Das System zur Durchführung von mindestens einer operativen Funktion gemäß dem vorausgegangenen Anspruch, **dadurch gekennzeichnet, dass** die Steuervorrichtungen jeweils Folgendes umfassen:
- Ultraschallerzeugerelemente (36) eines Typs, die geeignet sind, sowohl eine Druckwelle mit einem bestimmten Druck zu erzeugen, der geeignet ist, erste Platten (16, 17) der Trägervorrichtungen (10) zu bewegen und/oder zu perforieren, wodurch die anzuwendende, im Speicherbecken (15) enthaltene Substanz abgegeben wird, wie einen Ultraschallschalter (23c) zu betätigen;
- Laservorrichtungen (32), welche geeignet sind, eine Reihe von Lichtstrahlen mit bestimmten Emissionsfrequenzen für die vereinzelte oder simultane Betätigung von einem oder mehreren elektrischen Schaltern (23, 23b) abzugeben, wobei die Lichtstrahlen, welche durch plasmonische Filter (24) gefiltert werden, jeweils auf die genannten Emissionsfrequenzen geeicht sind.

## Revendications

1. Dispositif de support polyfonctionnel constitué de matériau biocompatible et sensiblement biodégradable, apte à être transporté dans des fluides et/ou des environnements organiques pour réaliser au moins une fonction opérationnelle, comprenant :
- un corps principal (12), constitué de matériau biocompatible et biodégradable, en particulier apte à constituer un réservoir (15) pour une substance à administrer et muni d'au moins une ouverture (13,14) pour l'écoulement d'une substance à administrer vers lesdits fluides et/ou environnements organiques ;
- des moyens d'actionnement (16,17 ; 21-25, 40,+,-) d'au moins une fonction du support polyfonctionnel (10) dans des fluides et/ou des environnements organiques,
**caractérisé en ce qu'**il comprend des moyens magnétiques (30,31), associés au corps principal (12), sous forme d'une pluralité d'éléments magnétiques d'un premier type (30) et d'une pluralité d'éléments magnétiques d'un second type (31) ayant respectivement une polarité sud/nord et nord/sud orientée vers le haut par rapport au corps principal (12), aptes à prendre en charge une polarité magnétique définie pour guider et positionner ledit support (10) dans des fluides et/ou des environnements organiques au moyen de champs magnétiques opérant à l'extérieur desdits fluides et/ou environnements organiques, et sont disposés le long des surfaces extérieures (13,14) du corps principal (12), aptes globalement à générer un champ magnétique défini, polarisé et dirigé afin d'agréger une pluralité de dispositifs de support (10) dans une configuration spatiale définie par lesdits moyens magnétiques (30,31).

2. Dispositif de support polyfonctionnel, selon la revendication précédente, **caractérisé en ce que** le corps principal (12) est formé de manière à avoir une forme extérieure apte à permettre l'agrégation de plusieurs dispositifs de support (10) en contact réciproque au moins avec une face.

3. Dispositif de support polyfonctionnel, selon l'une des revendications précédentes, **caractérisé en ce qu'**il comprend des moyens de fourniture et/ou stockage d'une charge électrique (21,22) associés audit corps principal (12), les moyens d'actionnement d'au moins une fonction (21-25,40,+,-) étant associés au corps principal (12) et auxdits moyens de fourniture et/ou stockage d'une charge électrique (21,22) afin de réaliser ladite au moins une fonction dans les fluides et/ou les environnements organiques où ils fonctionnent.

4. Dispositif de support polyfonctionnel, selon la revendication précédente, **caractérisé en ce qu'**il comprend au moins une surface de support (20), associée au corps principal (12), pour des moyens et des dispositifs aptes à mettre en oeuvre au moins une fonction opérationnelle du dispositif de support polyfonctionnel (10) auquel lesdits moyens de fourniture et/ou stockage d'une charge électrique (21,22) et/ou lesdits moyens pour actionner au moins une fonction (21-25,40,+,-) sont associés.

5. Dispositif de support polyfonctionnel, selon l'une des revendications 3 à 4, **caractérisé en ce que** les moyens d'actionnement d'au moins une fonction comprennent, sélectivement ou conjointement, les dispositifs et les moyens suivants :
- une DELO en matière organique (25) avec la fonction de tracer et détecter la position ;
- une paire de polarité (+,-) obtenue sur le corps principal (12) du dispositif de support (10) consistant en matière organique afin de transporter une fonction de stimulation électrique vers certaines parties de l'environnement organique dans lequel les dispositifs de support (10) fonctionnent ;
- un dispositif d'ouverture (40) pour ouvrir le réservoir (15) ;
lesdits dispositifs étant commandés :
- par un commutateur électrique (23a,23b) fait de matériau biocompatible et fonctionnant au moyen de l'énergie d'un faisceau laser (32) filtrée par un filtre plasmonique (24), le commutateur électrique (23a, 23b) étant apte à passer à la position fermée lorsqu'il est activé par la chaleur générée par le faisceau laser incident à une fréquence d'émission donnée à laquelle ledit filtre (24) est étalonné ou ;
- par un commutateur à ultrasons (23c) qui peut fonctionner au moyen d'ultrasons.

6. Dispositif de support polyfonctionnel, selon l'une des revendications 4 à 5, **caractérisé en ce qu'**il comprend des moyens de criblage et de protection contre la lumière appliquée au-dessus desdites surfaces de support (20) et des moyens et dispositifs relatifs aptes à mettre en oeuvre au moins une fonction opérationnelle du dispositif de support polyfonctionnel (10) associé à ceux-ci, en particulier lesdits moyens de fourniture et/ou stockage d'une charge électrique (21,22) et/ou lesdits moyens pour réaliser au moins une fonction (21-25,40,+,-), lesdits moyens de criblage et de protection contre la lumière n'étant pas présents au-dessus d'aucun des filtres plasmoniques (24) présents sur les surfaces de support (20) .

7. Système pour réaliser au moins une fonction opérationnelle dans des fluides et/ou des environnements organiques, **caractérisé en ce qu'**il comprend :
- au moins un dispositif de support polyfonctionnel (10) selon l'une des revendications précédentes ;
- des moyens d'injection (38) pour injecter des dispositifs de support polyfonctionnel (10) dans des fluides et/ou des environnements organiques ;
- des moyens de génération pour générer un champ magnétique (34,35) d'un type apte à diriger les dispositifs de support polyfonctionnel (10) dans les fluides et/ou les environnements organiques pour le fonctionnement selon des trajets spatiaux donnés et souhaités ;
- des dispositifs de commande (36;32,36) pour réaliser au moins une fonction du support polyfonctionnel (10) dans des fluides et/ou des environnements organiques.

8. Système pour réaliser au moins une fonction opérationnelle, selon la revendication précédente, **caractérisé en ce que** les dispositifs de commande comprennent :
- des moyens de génération d'ultrasons (36) d'un type apte à la fois à créer une onde de choc ayant une certaine pression apte à déplacer et/ou perforer des premières feuilles (16,17) des dispositifs de support (10) en libérant ainsi la substance à administrer contenue dans le réservoir (15), et à activer un commutateur à ultrasons (23c) ;
- des dispositifs laser (32) aptes à émettre une série de faisceaux lumineux à des fréquences d'émission données pour l'activation isolée ou simultanée d'un ou de plusieurs commutateurs électriques (23a, 23b), les faisceaux lumineux étant filtrés par des filtres plasmoniques (24) étalonnés sur lesdites fréquences d'émission.
